# EUROPEAN PATENT SPECIFICATION

(11) **EP 1 682 546 B1**
(45) Date of publication and mention of the grant of the patent: **03.06.2009**
(21) Application number: 04769699.2
(22) Date of filing: 22.10.2004
(51) Int. Cl.: C07D 471/04, C07K 5/00, A61K 31/437, A61K 38/04, A61P 13/00, A61P 19/10, A61P 35/04

(54) **PEPTIDOMIMETIC COMPOUNDS, STEREOSELECTIVE PROCESS FOR THEIR PREPARATION, THEIR USE AS BIOLOGICALLY ACTIVE SYNTHETIC INTERMEDIATES**
PEPTIDOMIMETISCHE VERBINDUNGEN, STEREOSELEKTIVES VERFAHREN ZU DEREN HERSTELLUNG UND DEREN VERWENDUNG ALS BIOLOGISCH WIRKSAME SYNTHESEZWISCHENPRODUKTE
COMPOSES PEPTIDOMIMETIQUES, LEUR PROCEDE STEREOSELECTIF DE PREPARATION, ET LEURS APPLICATIONS COMME INTERMEDIAIRES DE SYNTHESE A ACTIVITE BIOLOGIQUE

(30) Priority: 30.10.2003 IT MI20032102
(43) Date of publication of application: 26.07.2006
(73) Proprietor: CISI Scarl, 20138 Milano (MI) (IT)
(72) Inventor: SCOLASTICO, Carlo, Dip. di Chimica Organica e Ind., I-20133 Milan (IT); MANZONI, Leonardo, C.N.R. - ISTM, I-20133 Milan (IT); COLOMBO, Matteo, Dip. di Chimica Organica e Ind., I-20133 (IT); BRACCI, Antonio, Dip. di Chimica Organica e Ind., I-20133 Milan (IT)
(74) Representative: Trupiano, Federica
(86) International application number: PCT/IB2004/003464
(87) International publication number: WO 2005/042531

(56) References cited:
- EP-A- 1 077 218
- WO-A-20/04046173
- ARTALE E ET AL: "Synthesis of substituted conformationally constrained 6,5- and 7,5-fused bicyclic lactams as dipeptide mimics" TETRAHEDRON, ELSEVIER SCIENCE PUBLISHERS, AMSTERDAM, NL, vol. 59, no. 33, 11 August 2003 (2003-08-11), pages 6241-6250, XP004444194 ISSN: 0040-4020
- BELVISI L ET AL: "POTENT INTEGRIN ANTAGONISTS FROM A SMALL LIBRARY OF RGD-INCLUDING CYCLIC PSEUDOPEPTIDES" ORGANIC LETTERS, ACS, WASHINGTON, DC, US, vol. 3, no. 7, 2001, pages 1001-1004, XP001037188 ISSN: 1523-7060 cited in the application
- COLOMBO ET AL.: "Conformationally constrained dipeptides: synthesis of bicyclic lactams by stereoselective radical cyclizations" GAZZETTA CHIMICA ITALIANA, vol. 126, 1996, pages 543-554, XP009044041 cited in the application

## Description

The present invention relates to novel cyclic peptidomimetic compounds having an aza-bicycloalkanic structure. Said compounds are useful as intermediates in the preparation of αvβ3 and αvβ5 integrin antagonists which are useful, for example, in the treatment of altered angiogenic phenomena. The invention also concerns a process for the stereoselective synthesis of said cyclic peptidomimetic compounds and biologically active derivatives thereof.

### BACKGROUND OF THE INVENTION

A great number of physiological processes involve biologically active peptides, through their interactions with receptors and enzymes. However, peptides are not to be considered ideal drugs, given their poor metabolic stability, rapid excretion and low selectivity for specific receptors. A valid alternative involves the design of peptide analogues which are capable of mimicking the action of the native peptide at the receptor level (peptidomimetic) [(a) Kahn, M. (Editor). Peptide Secondary Structure Mimetics. Tetrahedron Symposia-in-Print No. 50 1993, 49, 3433-3689. (b) Gante, J. Angew. Chem., Int. Ed. Engl. 1994, 33, 1699-1720. (c) Olson, G. L.; Bolin, D. R.; Bonner, M. P.; Bös, M.; Cook, C. M.; Fry, D. C.; Graves, B. J.; Hatada, M.; Hill, D. E.; Kahn, M.; Madison, V. S.; Rusiecki, V. K.; Sarabu, R.; Sepinwall, J.; Vincent, G. P.; Voss, M. E. J. Med. Chem. 1993, 36, 3039-3049. (d) Kitagawa, O.; Velde, D. V.; Dutta, D.; Morton, M.; Takusagawa, F.; Aubè, J. J. Am. Chem. Soc. 1995, 117, 5169-5178. (e) Giannis, A.; Kolter, T. Angew. Chem.; Int. Ed. Engl. 1993, 32, 1244. (f) Aube, J. Tetrahedron Symposia-in-Print No. 50, 2000, 56, 9725-9842].

During the course of our research on peptide secondary structure mimetics, several 6,5- and 7,5-aza-bicycloalkanic amino acids have been synthesised [(a) Lino Colombo, Marcello Di Giacomo, Carlo Scolastico, Leonardo Manzoni, Laura Belvisi, Valentina Molteni, Tetrahedron Lett. 1995, 36, 625; (b) Colombo, L.; Di Giacomo, M.; Belvisi, L.; Manzoni, L.; Scolastico, C. Gazz. Chim. It. 1996, 126, 543; (c) Colombo, L.; Di Giacomo, M.; Brusotti, G.; Sardone, N.; Angiolini, M.; Belvisi, L.; Maffioli, S.; Manzoni, L.; Scolastico, C. Tetrahedron 1998, 54, 5325-5336; (d) Angiolini, M.; Araneo, S.; Belvisi, L.; Cesarotti, E.; Checchia, A.; Crippa, L.; Manzoni, L.; Scolastico, C. Eur. J. Org. Chem. 2000, 2571-2581; (e) Manzoni, L.; Colombo, M.; May, E.; Scolastico, C. Tetrahedron 2001, 57, 249; (f) Belvisi, L.; Colombo, L.; Colombo, M.; Di Giacomo, M.; Manzoni, L.; Vodopivec, B.; Scolastico, C. Tetrahedron 2001, 57, 6463; (g) EP 1 077 218.].

These structures may be considered as conformationally constrained analogues of the Ala-Pro and Phe-Pro dipeptide units. [(a) Belvisi, L.; Bernardi, A.; Manzoni, L.; Potenza, D.; Scolastico, C. Eur. J. Org. Chem. 2000, 2563-2569; (b) Gennari, C.; Mielgo, A.; Potenza, D.; Scolastico, C.; Piarulli, U.; Manzoni, L. Eur. J. Org. Chem. 1999, 379].

The functionalisation of such molecules with heteroalkyl substituents is an aim of great interest, since the side chains may increase the affinity of the peptide for the receptor by interacting with the hydrophobic or hydrophilic pockets of the receptor itself. A further advantage of such systems is the potential for binding to different pharmacophoric groups and hence the possibility of generating a library, the members of which having different properties and biological activities.

Furthermore, analogously to that which occurs for the non-substituted conformationally constrained dipeptide mimetics [Belvisi, L.; Bemardi, A.; Checchia, A.; Manzoni, L.; Potenza, D.; Scolastico, C.; Castorina, M.; Cupelli, A.; Giannini, G.; Carminati, P.; Pisano, C. Org. Lett. 2001, 3, 1001] such heteroalkyl substituted lactams may be incorporated into cyclic pseudo-peptides containing the sequence RGD. Such molecules may be selectively targeted to those tissues over-expressing certain receptors (*e*.*g*. epithelial cells involved in vascular growth), so as to be able to be used for the inhibition of angiogenesis and selectively control the release of any drugs optionally bound to the substituents present on the lactamic system [Arap, W.; Pasqualini, R.; Ruoslahti, E. Science, 1998, 279, 377].

The low number of "scaffolds" reported in the literature necessitates the design and synthesis of novel conformationally constrained dipeptide mimetics functionalised by hetero-substituted side chains for interaction with various receptors.

### DESCRIPTION OF THE INVENTION

It has been found that certain conformationally constrained aza-bicyclic[X.Y.0]alkanes satisfy the features required for the application of this type of technology. Particularly, it has now been found that compounds having the conformationally constrained homoSer-Pro dipeptide unit structure are useful as drugs, particularly as drugs with antagonistic action towards the αvβ3 and αvβ5 integrins.

Thus, according to one of the aspects thereof, the invention relates to compounds of general formula (I) 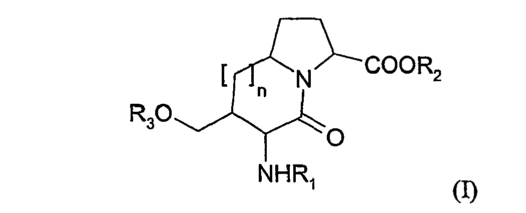 wherein:
n is 1 or 2,
R₁ is H, (C₁-C₄)alkyl, benzyl or a protective group;
R₂ is H or a protective group;
R₃ is H, heteroalkyl or a protective group; their salts, racemic mixtures, individual enantiomers, individual diastereoisomers and mixtures thereof in any proportion.

According to one advantageous aspect, the object of the present invention are the compounds of formulae (Ia) and (Ib) 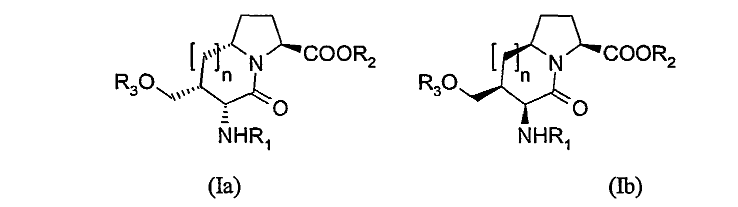 wherein n, R₁, R₂ and R₃ are as defined above and the wedge-shaped and dotted bonds indicate that the substituents are positioned above and below the plane respectively.

According to the present invention, the term "(C₁-C₄)alkyl" designates a linear or branched, saturated or unsaturated alkyl substituent comprising 1 to 4 carbon atoms such as for example methyl, ethyl, propyl, isopropyl, butyl, tert-butyl However, it is possible to use alkyl substituents containing a higher number of carbon atoms providing they are compatible with the reaction conditions of the present invention.

According to the present invention, the term "heteroalkyl" designates a saturated or unsaturated, linear or branched aliphatic, containing at least one heteroatom, for example nitrogen, oxygen or sulphur. Preferred heteroalkyl substituents are saturated alkyl chains substituted with OH, NH₂ or SH functions. Most preferred heteroalkyl substituents are -(CH₂)ₘCH₂OH, -(CH₂)ₘCH₂NH₂, -(CH₂)ₘCH₂SH, chains wherein m is a number between 0 and 10, advantageously between 0 and 6.

According to the present invention, the expression "protective group" designates a protective group suitable to preserve the function to which it is bound, specifically the amino or hydroxyl function. Appropriate protective groups are for example benzyl, alkyl or benzyl esters, or other substituents routinely used for the protection of such functions, which are well known to those skilled in the art, for example those described in conventional manuals such as T. W. Green, Protective Groups in Organic Synthesis (Wiley, N.Y. 1981).

The salts of the compounds of formulae (I), (Ia) and (Ib) according to the present invention comprise both those with mineral or organic acids which allow the separation or expedient crystallisation of the compounds of the invention, and those which form physiologically and pharmaceutically acceptable salts, such as for example hydrochloride, hydrobromide, sulphate, hydrogen sulphate, dihydrogen sulphate, maleate, fumarate, 2-naphthalenesulphonate, para-toluenesulphonate, oxalate etc. Said salts are prepared according to techniques well known to those skilled in the art.

When a free carboxyl group (R₂=H) is present, the salts of the compounds of the invention also comprise salts with organic or mineral bases, such as for example alkaline metal or alkaline earth metal salts, such as salts of sodium, potassium or calcium, or amine salts such as trometamol (tromethamine), or salts of arginine, lysine or any other physiologically and pharmaceutically acceptable amine.

According to one preferred embodiment, the object of the present invention are the compounds of formulae (I), (Ia) and (Ib) wherein n is 1, R₁ is H, R₂ is tert-butyl and R₃ is H.

According to another preferred embodiment, the object of the present invention are the compounds of formulae (I), (Ia) and (Ib) wherein n is 2, R₁ is H, R₂ is tert-butyl and R₃ is H.

According to another aspect thereof, the object of the present invention is the stereoselective synthesis of compounds of general formulae (I), (Ia) and (Ib) as defined previously.

A process for the stereoselective synthesis of such compounds is described in detail over the course of the present description, making reference to the synthetic outline reported in the following Figures.

Figure 1 shows a general outline for the synthesis of compounds of formulae (Ia) (*trans* series) and (Ib) (*cis* series).

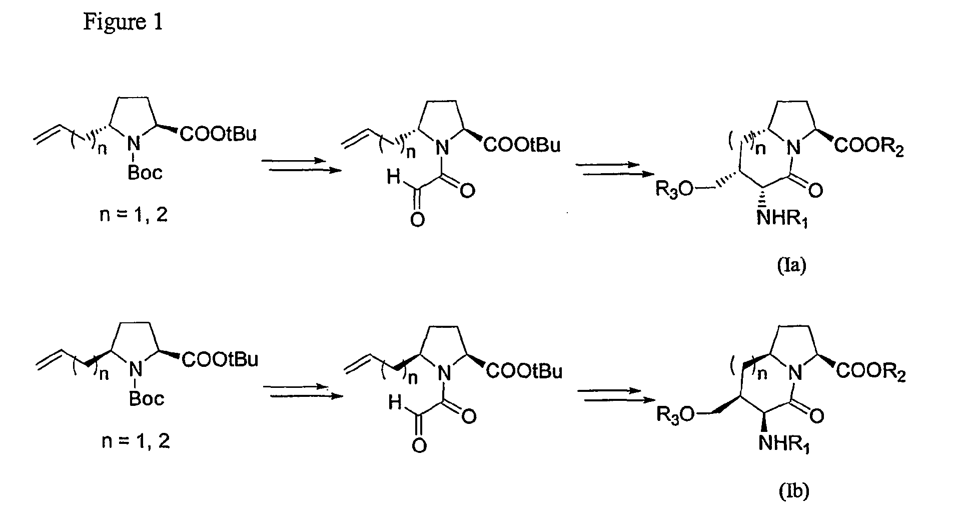

Figure 2 shows the synthesis of the homoalkyl compounds **7** (*trans*) and **8** (*cis*). 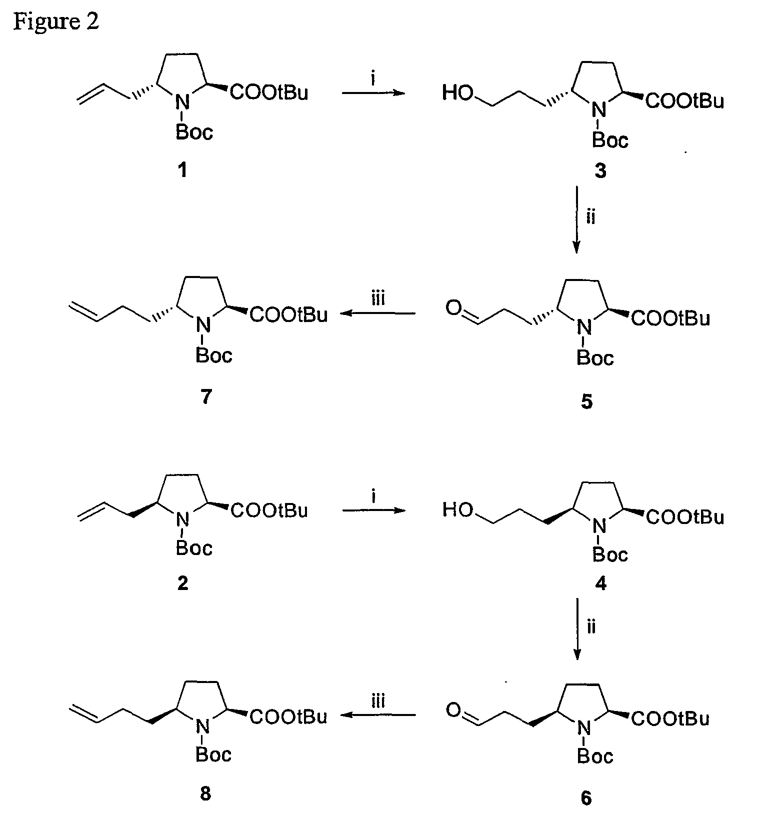
i. 9-BBN, THF, 0°C; ii. (COCl)₂, DMSO, TEA, CH₂Cl₂, -60°C; iii. Ph₃PCH₃Br, BuLi, THF, -78°C 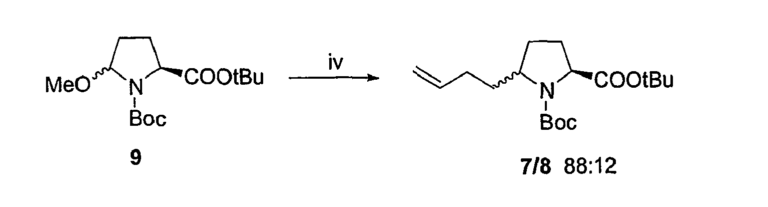
iv. CH₂=CHCHCHMgBr, CuBr·DMS, BF₃·Et₂O, THF, -78°C

Figure 3 shows the synthesis of the *trans*-condensated bicyclic lactams of general formula (Ia). 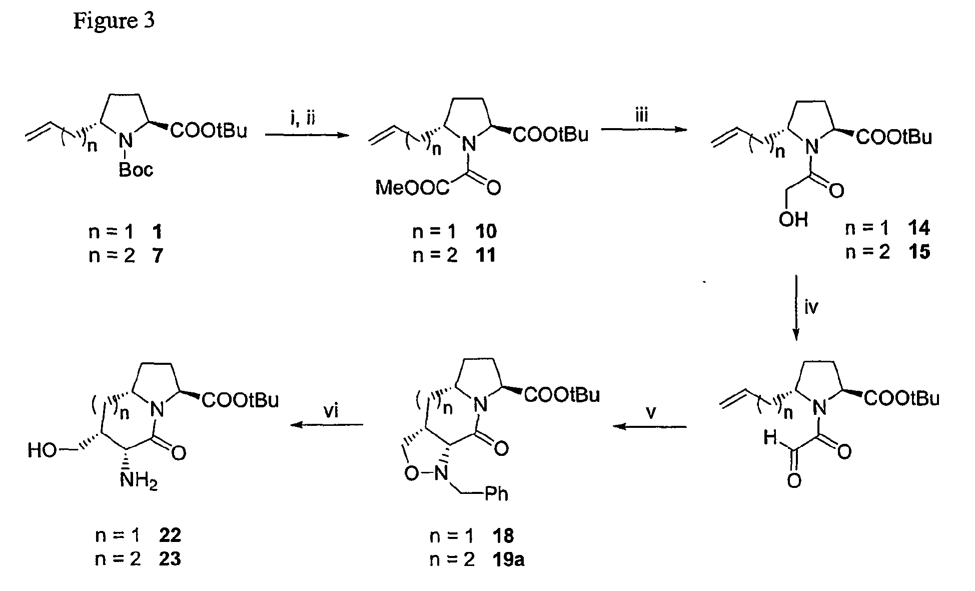
i. HClO₄, AcOtBu, 0°C; ii. Amberlyst A-21, ClCOCOOMe, -20°C; iii. LiBH₄, THF, 0°C; iv. (COCl)₂, DMSO, TEA, CH₂Cl₂, -60°C; v. Bn-NH-OH-HCl, NaHCO₃, EtOH/H₂O, 80°C; vi. H₂, Pd/C, MeOH

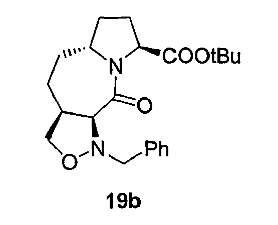

Figure 4 shows the synthesis of the *cis*-condensated bicyclic lactams of general formula (Ib). 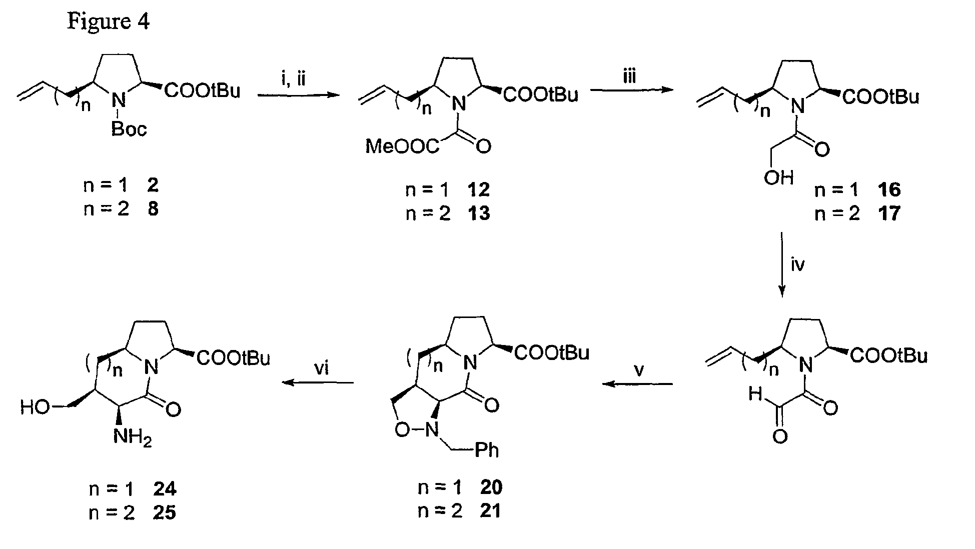
i. HClO₄, AcOtBu, 0°C; ii. Amberlyst A-21, ClCOCOOMe, -20°C; iii. LiBH₄, THF, 0°C; iv. (COCl)₂, DMSO, TEA, CH₂Cl₂, -60°C; v. Bn-NH-OH-HCl, NaHCO₃, EtOH/H₂O, 80°C; vi. H₂, Pd/C, MeOH

In accordance with the present invention, the compounds of formulae (I), (Ia) and (Ib) may be prepared according to the processes described below.

Particularly, the compounds of general formulae (Ia) *6*,*5-trans-* and (Ib) 6,5-*cis*-condensated, may be prepared according to a synthetic process outlined in Figures 3 and 4 (wherein n=1), which comprises:
a) the chemoselective deprotection of the nitrogen of compound (1) of Figure 3 or of compound (2) of Figure 4 and condensation with oxalic acid monomethyl ester (or with an acyl chloride thereof) followed by the chemoselective reduction of the methyl ester to alcohol to give the compounds of formulae **14, 16;**
b) the oxidation to aldehyde and subsequent formation of the nitrone by condensation with N-benzylhydroxylamine, involved in the 1,3-dipolar reaction, followed by catalytic hydrogenation to give the compounds of general formula (I).

The compounds of general formulae (Ia) 7,5-*trans-* and (Ib) *7,5-cis-*condensated, may be prepared according to a synthetic process outlined in Figure 2 (step (a)) and in Figures 3 and 4 (wherein n=2) (steps (b) and (c)) comprising:
a) the hydroboration of the allyl compound (1) of Figure 2, followed by oxidation of the hydroxyl group thus formed and the subsequent Wittig-reaction mediated methylation to give the compounds of formulae **7**, **8**, or the introduction of the homoallyl residue by the organo-copper mediated alkylation of compound **9**;
b) the chemoselective deprotection of the nitrogen of compound (7) of Figure 3 or of compound (8) of Figure 4 and condensation with oxalic acid monomethyl ester (or with the acyl chloride thereof) followed by the chemoselective reduction of the methyl ester to alcohol to give the compounds of formulae **15, 17**;
c) the oxidation to aldehyde and subsequent formation of the nitrone involved in the 1,3-dipolar reaction followed by catalytic hydrogenation to give the compounds of general formula (I).

The synthetic process for the preparation of the compounds of formulae (I), (Ia) and (Ib) is hence substantially based on an intramolecular 1,3-dipolar cycloaddition reaction between a double bond and a nitrone.

Following hydrogenation, the resulting lactams possess a hydroxymethyl group, to which appropriate substituents or spacer groups may be bound, within the reach of those skilled in the art.

The allyl (n=1) starting compounds may be prepared according to the previously described procedures [Angiolini, M.; Araneo, S.; Belvisi, L.; Cesarotti, E.; Checchia, A.; Crippa, L.; Manzoni, L.; Scolastico, C. Eur. J. Org. Chem. 2000, 2571-2581].

The homoallyl (n=2) starting compounds may be prepared as reported in the present description.

Said homoallyl starting products have been synthesised from the corresponding allyl derivatives using the synthetic process reported in Figure 2. Hydroboration of the double bond by treatment with 9-BBN in THF has resulted in the corresponding alcohols **3, 4** which, once oxidised by the Swern reaction, have provided the corresponding aldehydes **5, 6**. Methylation of said aldehydes has been performed by the Wittig reaction, thus giving products **7, 8**.

Alternatively, and more advantageously, the *trans* homoallyl products may also be synthesised by the alkylation of compound 9 with 3-butenyl-magnesium bromide, CuBr and BF₃·Et₂O.

Examples of such preparations are reported in the experimental section of the present description.

Synthesis of the lactams **22, 23** (*trans*-condensated) has been carried out according to the outline reported in Figure 3. The corresponding olefin, chemoselectively deprotected at the nitrogen by treatment with HClO₄ in AcOtBu, provides the corresponding free amine, which, by condensation with the commercially available chloride salt of oxalic acid monomethyl ester, leads to the formation of products **10** and **11**. The chemoselective reduction of the methyl ester to the alcohol has been performed with high yield, by treatment with LiBH₄ in THF, the alcohol thus obtained has been oxidised to the corresponding aldehyde by the Swern procedure, which, following treatment with N-benzyl hydroxylamine, has led to the formation of the tricyclic compounds **18, 19** through a intramolecular 1,3-dipolar cycloaddition reaction. Catalytic hydrogenation of compounds **18** and **19** over Pd/C provides the corresponding aminoalcohols **22** and **23.**

A process, entirely analogous to that used for the synthesis of the compounds reported in Figure 3, may be used for the synthesis of compounds **24, 25** (*cis-*condensated) reported in Figure 4.

The stereochemistry of the stereocentres which form over the course of the cycloaddition reaction has been determined by NOE experiments on the tricyclic compounds **18-21**.

The reactions reported in the outlines in the enclosed Figures are described in detail, by way of exemplification, in the experimental part of the present description.

The compounds of the present invention of formulae (I), (Ia) and (Ib) may be seen as conformationally constrained scaffolds with the potential to replicate the geometry of the backbone and the side chains of a dipeptide residue within the active site. These compounds may be used as conformationally constrained entities which mimic segments of native peptides.

Another application of the present invention is the use of the compounds as "reverse-turn" inducers and as "scaffolds" for the synthesis of biologically active compounds.

The compounds of formula (I) as defined above, advantageously those of formulae (Ia) and (Ib), are indeed synthetic intermediates, particularly useful for the preparation of cyclic peptides, particularly peptides containing the sequence Arg-Gly-Asp (RGD). The substitution of the R₁ and R₂ groups with the Arg-Gly-Asp (RGD) chain, indeed allows the attainment of cyclic peptide compounds with high biological activity.

Thus, the present invention concerns, according to another aspect thereof, the use of the compounds of formulae (I), (Ia) and (Ib) as synthetic intermediates in the preparation of cyclic peptides, advantageously cyclic peptides wherein R₁ and R₂ together form a peptide sequence, preferably the sequence Arg-Gly-Asp (RGD).

The compounds of formulae (I), (Ia) and (Ib) may hence be modified in order to give antiangiogenic activity. With reference to antiangiogenic treatment, a useful example may be that reported in the patent EP 1 077 218.

The compounds of formulae (I), (Ia) and (Ib) wherein R₁ and R₂ together represent the sequence RGD are integrin inhibitors, and particularly they are selective inhibitors for αvβ3 and αvβ5 integrins. Said compounds are indicated below by the general formula (II).

Thus, in accordance with another aspect thereof, the invention has as a subject, novel compounds of formula (II) 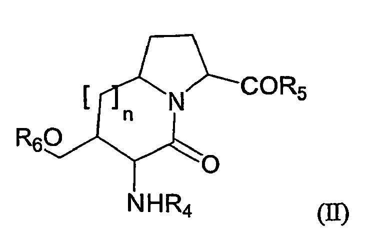 wherein:
n is 1 or 2,
R₄ and R₅ together form the sequence Asp-Gly-Arg,
R₆ is H or heteroalkyl or a protective group;
their salts, racemic mixtures, individual enantiomers, individual diastereoisomers and mixtures thereof in any proportion.

According to one preferred aspect, the invention concerns compounds of formulae (IIa) and (IIb) 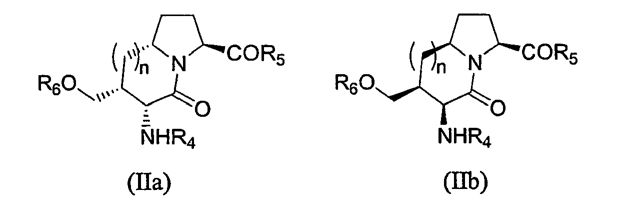 wherein n, R₄, R₅ and R₆ are as defined above and the wedge-shaped and dotted bonds indicate that the substituents are positioned above and below the plane respectively.

The sequence Asp-Gly-Arg is advantageously bound to compounds (II), (IIa) and (IIb) in such a manner whereby the carboxyl group is attached to the amino acid arginine and the amino group is attached to the amino acid aspartic acid, by conventional peptide bonds, *i*.*e*. the compounds of formula

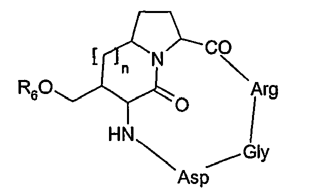

The details provided above for the variable substituents (alkyl, etc.) and the salts of the compounds of formula (I) are also applicable to the compounds of formulae (II), (IIa) and (IIb).

The compounds of formulae (II), (IIa) and (IIb) may be prepared by starting from the compounds of formulae (I), (Ia) and (Ib) described above, according to a process involving the following steps:
- chemoselective deprotection reaction of the carboxyl group of the compound of general formula (I) and condensation with the appropriately protected and previously prepared Arg-Gly dipeptide;
- chemoselective deprotection of the amino group of aza-bicycloallcane and subsequent condensation with appropriately protected aspartic acid;
- transformation of the methyl ester of glycine into the benzyl ester through a transesterification reaction, followed by the simultaneous removal of the protective group from the glycine and the amino group from the aspartic acid by catalytic hydrogenation;
- condensation agent mediated intramolecular cyclisation and subsequent: deprotection of the amino acid side chain protective groups.

The functional group protection and deprotection reactions may be carried out according to techniques known to those skilled in the art.

Details of this preparation on compounds structurally analogous to those of the present invention are provided in Italian patent application N° MI2003A 001476 filed on 18th July 2003 in the name of the present applicant.

The compounds of formulae (II), (IIa) and (IIb) possess interesting pharmacological properties, particularly an antagonistic effect towards the αvβ3 and αvβ5 integrins, and display interesting antiangiogenic activities.

A further subject of the present invention is hence the use of the compounds of general formulae (II), (IIa) and (IIb) for the preparation of drugs, particularly useful for their antagonistic action towards the αvβ3 and αvβ5 integrins.

More precisely, the invention concerns the use of compounds of general formulae (II), (IIa) and (IIb) for the preparation of drugs useful for the treatment of altered angiogenic phenomena, such as those encountered in metastasising tumour processes, retinopathies, acute renal damage and osteoporosis.

Biological tests for the evaluation of the activities of the compounds of general formulae (II), (IIa) and (IIb) towards the αvβ3 and αvβ5 integrins have been performed using tests known and described in the literature [C. C. Kumar, H. Nie, C. P. Rogers, M. Malkowski, E. Maxwell, J. J. Catino, L. Armstrong, J. Pharmacol. Exp. Ther. 1997, 283, 843] for example such as that reported in patent application EP 1077218.

In such tests, some compounds representative of the invention have shown interesting biological and pharmacological activities.

Regarding their use as drugs according to the invention, the compounds of general formulae (II), (IIa) and (IIb), their pharmaceutically acceptable salts, racemic mixtures, individual enantiomers, individual stereoisomers and mixtures thereof in any proportion, are advantageously formulated into pharmaceutical compositions according to conventional techniques, well known to those skilled in the art.

Thus, according to another aspect thereof, the invention concerns pharmaceutical compositions containing, as active ingredient, at least one compound of general formulae (II), (IIa) or (IIb), their pharmaceutically acceptable salts, racemic mixtures, individual enantiomers, individual diastereoisomers and mixtures thereof in any proportion, in combination with one or more possible pharmaceutically acceptable carriers or excipients.

The compositions of the invention may be prepared according to conventional techniques well known to those skilled in pharmaceutical technique.

In order to obtain a prophylactic or desired therapeutic effect, the dose of active ingredient is advantageously administered in the form of a unit dose, one or more times per day.

The compounds of formulae (II), (IIa) and (IIb) may be combined with various drugs, for example with a cytotoxic type drug, active towards the tumour pathology.

In fact, the functionalised side chain (-OR₆ substituent) of the compounds of the invention may be exploited as a site for the introduction of groups, relevant from the pharmacological viewpoint, in order to enhance protein-protein or protein-receptor interactions.

Thus, for example, in the compounds of formulae (II), (IIa) and (IIb) wherein R₆ represents a hydrogen atom, the hydroxymethyl group is available for the simplified conjugation of a drug. Alternatively, depending on the type of drug to be conjugated, R₆ may be more conveniently a heteroalkyl, as defined above.

According to another aspect thereof, the invention concerns the use of compounds of formulae (II), (IIa) and (IIb) as mediators for the transport and release of drugs.

Thus way it is possible to bind a drug, conventionally, through groups available for the formation of a chemical bond as outlined above, to the compounds of formulae (II), (IIa) and (IIb).

Thus, the drug conjugated to the compounds of formulae (II), (IIa) or (IIb) may then be transported to the desired site of action in order to perform its pharmacological activity.

The conjugates of the compounds of formulae (II), (IIa) or (IIb) as described above, with drugs, advantageously with cytotoxic and antitumour drugs, constitute another aspect of the present invention.

The present invention will now be described from the experimental viewpoint.

### EXPERIMENTAL SECTION

**General Observations:** The ¹H- and ¹³C-NMR spectra have been recorded in CDCl₃ as indicated, at 200 (or 300, 400) and 50.3 (or 75.4) MHz, respectively. Chemical shift values are indicated in ppm and the coupling constants in Hz. - Optical rotatory powers are measured using a Perkin-Elmer model 241 polarimeter. - Thin layer chromatography (TLC) is performed using Merck F-254 plates. Flash chromatography is performed using Macherey-Nagel 60, 230-400 mesh silica gel. Solvents are anhydrified in accordance with standard procedures and reactions requiring anhydrous conditions are carried out in an atmosphere of nitrogen or argon. Solutions containing the final products are anhydrified using Na₂SO₄, filtered, and concentrated under reduced pressure using a rotary evaporator.

### PREPARATION OF HOMOALLYL DERIVATIVES (COMPOUNDS (7) AND (8) FIGURE 2)

### Example 1: Hydroboration.

To a solution of allyl-derivative **1** or **2** (1,000 mg, 3.21 mmol) in anhydrous THF (32 ml) under an atmosphere of nitrogen and cooled to 0°C is added a 0.5M solution of 9-BBN in THF (25.7 ml, 12.82 mmol). The solution is stirred for 1 hour at room temperature, then phosphate buffer pH 7 (32 ml) and 35% H₂O₂ (3.2 ml) are added at 0°C and the mixture is kept stirring overnight. The solution is then extracted with AcOEt (3x30 ml), the organic phases combined and anhydrified over Na₂SO₄ and the solvent evaporated under reduced pressure. The crude product thus obtained is purified by flash chromatography (hexane/AcOEt 6:4) to give the alcohols **3** or **4**. Alcohol **3**: obtained as a white solid according to general procedure A (87% yield). m.p. = 97-99°C. [α]_{D}²² = -66.1 (*c* = 1.01, CHCl₃). ¹H NMR (200 MHz, CDCl₃) (mixture of conformers): δ 1.42, 1.47 (2 s, 18H, COO*tBu, Boc*), 1.5-2.35 (10H), 3.5 (m, 1H), 3.95-4.2 (2H, C*H*COOtBu, C*H*NBoc). ¹³C NMR (50.3 MHz, CDCl₃) (mixture of conformers): δ 172.0, 154.3, 154.0, 80.8, 80.7, 79.7, 79.4, 62.2, 61.8, 60.3, 60.2, 57.7, 57.4, 30.9, 30.2, 29.5, 29.1, 28.4, 28.2, 28.1, 27.8, 27.7, 27.4, 27.2. FAB⁺MS: calculated C₁₇H₃₁NO₅ 329.22, found 330 [M+1]⁺. Calculated elemental analysis C₁₇H₃₁NO₅: C 61.98, H 9.48, N 4.25; found C 62.03, H 9.65, N 4.44.
Alcohol **4**: obtained as a colourless oil according to general procedure A (93% yield). [α]_{D}²² = -20.2 (*c* = 1.01, CHCl₃). ¹H NMR (200 MHz, CDCl₃) (mixture of conformers): δ 1.41 (s, 18H, COO*tBu*, *Boc*), 1.42-2.23 (8H), 2.82 (s, 1H, CH₂O*H*), 3.6 (m, 2H, C*H*₂OH), 3.85-4.1 (2H, C*H*COOtBu and C*H*NBoc). ¹³C NMR (50.3 MHz, CDCl₃) (mixture of conformers): δ 172.3, 154.0, 82.3, 81.5, 81.3, 80.6, 79.7, 79.6, 62.3, 61.9, 60.5, 60.3, 60.2, 58.0, 57.6, 57.3, 30.6, 30.2, 30.1, 29.5, 29.1, 28.7, 28.2, 27.9, 27.8, 27.3, 23.7. FAB⁺MS: calculated C₁₇H₃₁NO₅ 329.22, found 330 [M+1]⁺. Calculated elemental analysis C₁₇H₃₁NO₅: C 61.98, H 9.48, N 4.25; found C 61.88, H 9.60, N 4.15.

### Example 2: Swern oxidation.

To a solution of (COCl)₂ (0.66 ml, 7.68 mmol) in anhydrous CH₂Cl₂ (17 ml) maintained in a nitrogen atmosphere and cooled to -60°C are added DMSO (0.73 ml, 10.24 mmol) and following 10 minutes, a solution of alcohol **3** or **4** (876.3 mg, 2.66 mmol) in anhydrous CH₂Cl₂ (9 ml). Anhydrous TEA (2.9 ml, 20.48) is added after 15 minutes and the temperature allowed to slowly rise. After 1 hour, phosphate buffer pH 7 (26 ml) is added and the solution is extracted with CH₂Cl₂ (3x25 ml), the organic phases are dried over Na₂SO₄ and the solvent evaporated. The crude product thus obtained is purified by flash chromatography (hexane/AcOEt 7:3) to give the aldehydes **5** or **6**.
Aldehyde **5**: obtained as a white solid according to general procedure B (98% yield). m.p. = 84-86°C. [α]_{D}²² = -68.2 (*c* = 1.00, CHCl₃). ¹H NMR (200 MHz, CDCl₃) (mixture of conformers): δ 1.43, 1.45, 1.47, 1.48 (4 s, 18H, COO*tBu*, *Boc*), 1.5-2.6 (8H), 3.9-4.2 (2H, C*H*N, C*H*COOtBu), 9.76 (m, 1H, CHO). ¹³C NMR (50.3 MHz, CDCl₃) (mixture of conformers): δ 201.8, 201.4, 171.9, 171.8, 154.0, 80.8, 79.8, 79.7, 60.5, 60.4, 57.1, 56.9, 40.9, 40.7, 28.5, 28.4, 28.2, 28.1, 27.8, 27.7, 27.5, 27.4, 27.1, 26.8. FAB⁺MS: calculated C₁₇H₂₉NO₅ 327.20, found 328 [M+1]⁺. Calculated elemental analysis C₁₇H₂₉NO₅: C 62.36, H 8.93, N 4.28; found C 62.52, H 8.77, N 4.16.
Aldehyde **6**: obtained as a colourless oil according to general procedure B (91% yield). [α]_{D}²² = -38.4 (*c* = 1.00, CHCl₃). ¹H NMR (300 MHz, CDCl₃) (mixture of conformers): δ 1.41 (s, 18H, COO*tBu, Boc*), 1.42-2.8 (8H), 3.8-4.3 (2H, C*H*COOtBu and *CH*NBoc), 9.8 (s, 1H, CHO). ¹³C NMR (50.3 MHz, CDCl₃) (mixture of conformers): δ 202.6, 172.6, 154.4, 81.1, 80.1, 60.9, 57.7, 57.4, 41.2, 32.3, 30.7, 30.5, 30.1, 29.1, 28.9, 28.5, 28.4, 28.2, 27.9, 27.1. FAB⁺MS: calculated C₁₇H₂₉NO₅ 327.20, found 328 [M+1]⁺. Calculated elemental analysis C₁₇H₂₉NO₅: C 62.36, H 8.93, N 4.28; found C 62.24, H 8.79, N 4.21.

### Example 3: Wittig olefination.

To a suspension of Ph₃PCH₃Br (360.1 mg, 1.008 mmol) in anhydrous THF (1.7 ml) under a nitrogen atmosphere cooled to 0°C is slowly added a 1.6M solution of BuLi in hexane (420 µl, 0.672 mmol). The yellow suspension thus obtained is stirred for 10 minutes at 0°C and 1 hour at room temperature, then a solution of aldehyde **5** or **6** (110.2 mg, 0.336 mmol) in anhydrous THF (1.7 ml) is added very slowly at -78°C. The mixture is slowly adjusted to room temperature and stirred for 1 hour, then H₂O (3.4 ml) is added and the mixture extracted with iPr₂O (3x3.5 ml). The organic phases are dried over Na₂SO₄ and evaporated; the crude product is purified by flash chromatography (Hexane/AcOEt 9:1) to give the olefins **7** or **8**.
Olefin **7**: obtained as a white solid according to general procedure C (79% yield). m.p. = 74-75°C. [α]_{D}²² = -68.6 (*c* = 1.00, CHCl₃). ¹H NMR (200 MHz, CDCl₃) (mixture of conformers): δ 1.43, 1.46 (2 s, 18H, COO*tBu, Boc*), 1.58-2.35 (8H), 3.93 (m, 1H, C*H*N), 4.16 (m, 1H, C*H*COOtBu), 4.99 (m, 2H, C*H*₂=CH), 5.82 (m, 1H, CH₂=C*H*). ¹³C NMR (50.3 MHz, CDCl₃) (mixture of conformers): δ 172.3, 172.2, 154.5, 153.9, 138.3, 138.1, 114.8, 114.6, 80.8, 79.6, 60.5, 57.9, 57.5, 34.0, 33.1, 31.0, 30.9, 28.7, 28.5, 28.4, 28.3, 28.1, 28.0, 27.7, 27.2. FAB⁺MS: calculated C₁₇H₃₁NO₄ 325.23, found 326 [M+1]⁺. Calculated elemental analysis C₁₈H₃₁NO₄: C 66.43, H 9.60, N 4.30; found C 66.53, H 9.48, N 4.18.
Olefin **8**: obtained as a colourless oil according to general procedure C (90% yield). [α]_{D}²² = -27.1 (*c* = 0.99, CHCl₃). ¹H NMR (200 MHz, CDCl₃) (mixture of conformers): δ 1.41, 1.43 (2 s, 18H, *Boc*, COO*tBu*), 1.78-2.25 (8H), 3.85, 4.07 (2 m, 2H, *CH*NCHCO, C*H*COOtBu), 4.95 (m, 2H, C*H*₂=CH), 5.80 (m, 1H, CH₂=C*H*). ¹³C NMR (50.3 MHz, CDCl₃) (mixture of conformers): δ 172.6, 154.0, 138.6, 138.4, 114.7, 114.6, 80.9, 80.8, 79.7, 79.6, 60.9, 60.6, 58.4, 58.1, 58.0, 57.6, 34.0, 33.8, 33.3, 33.2, 31.1, 30.1, 29.8, 29.4, 29.1, 28.8, 28.6, 28.5, 28.3, 28.2, 28.1, 27.8, 27.2. FAB⁺MS: calculated C₁₈H₃₁NO₄ 325.23, found 326 [M+1]⁺. Calculated elemental analysis C₁₈H₃₁NO₄: C 66.43, H 9.60, N 4.30; found C 66.29, H 9.71, N 4.38.

### Example 4: Organo-copper mediated alkylation.

To a suspension of Mg powder (359.2 mg, 14.78 mmol) in anhydrous THF (4.9 ml) under an atmosphere of argon are added a few crystals of I₂ and 4-bromo-1-butene (500 µl, 4.93 mmol), very slowly, given that the reaction is exothermic. After 1.5 hours at room temperature, the solution thus obtained is slowly added to a suspension of CuBr·DMS (814.7 mg, 3.96 mmol) in anhydrous THF (8 ml) under an atmosphere of argon and cooled to -78°C. After 45 minutes, BF₃·Et₂O (502 µl, 3.96 mmol) is added followed, after a further 30 minutes, by a solution of aminal **9** (298.6, 0.99 mmol) in anhydrous THF (2 ml). After 15 minutes at -78°C, the solution is allowed to return to room temperature, treated with a 1:1 saturated NH₄Cl/NH₄OH solution (20 ml), left stirring for 16 hours and then extracted with iPr₂O (4x35 ml). The organic phases are washed with saturated NaHCO₃ (15 ml), dried over Na₂SO₄, filtered and evaporated. The crude product thus obtained is purified by flash chromatography (hexane/AcOEt 93:7) to give compounds **7** and **8** (306.3 mg, yield 95%) in the *trans*/*cis* ratio of 88:12.

### PREPARATION OF THE COMPOUNDS OF FORMULAE (Ia) AND (Ib)

### Example 5: Acylation of the allyl-derivatives.

To a solution of allyl **1, 2, 7** or **8** (0.292 mmol) in AcOtBu (2.9 ml), cooled to 0°C, is slowly added 70% HClO₄ (75 µl, 0.876 mmol). After 2 hours, basic Amberlyst A-21 resin (620 mg) is added along with CH₂Cl₂ (2.9 ml) in order to assist agitation. The mixture is then cooled to -20°C and ClCOCOOMe (54 µl, 0.584 mmol) is added slowly, dropwise. Upon reaching room temperature, the suspension is filtered and washed with CH₂Cl₂ (15 ml). The organic phase is evaporated and the crude product purified by flash chromatography (hexane/AcOEt 75:25) to give the methyl esters **10, 11, 12** or **13**.

Methyl ester **10**: obtained as a colourless oil according to general procedure E (76% yield, 2 steps). [α]_{D}²² = -104.2 (*c* = 1.00, CHCl₃). ¹H NMR (200 MHz, CDCl₃) (mixture of conformers): δ 1.41 (s, 9H, COO*tBu*), 1.72-2.62 (6H), 3.77, 3.83 (2 s, 3H, COO*Me*), 4.40 (m, 1H, C*H*N), 4.81 (m, 1H, C*H*COOtBu), 5.04 (m, 2H, C*H*₂=CH), 5.69 (m, 1H, CH₂=C*H*). ¹³C NMR (50.3 MHz, CDCl₃) (mixture of conformers): δ 171.3, 170.1, 162.3, 158.4, 134.6, 133.9, 118.6, 117.9, 82.4, 81.9, 61.8, 60.7, 59.2, 58.7, 52.9, 40.3, 36.8, 29.4, 28.5, 28.0, 27.9, 26.5, 26.0. FAB⁺MS: calculated C₁₅H₂₃NO₅ 297.16, found 298 [M+1]⁺. Calculated elemental analysis C₁₅H₂₃NO₅: C 60.59, H 7.80, N 4.71; found C 60.39, H 7.94, N 4.63.

Methyl ester **11:** obtained as a colourless oil according to general procedure E (80% yield, 2 steps). [α]_{D}²² = -105.7 (*c* = 1.00, CHCl₃). ¹H NMR (200 MHz, CDCl₃) (mixture of conformers): δ 1.44 (s, 9H, COO*tBu),* 1.78-2.32 (8H), 3.82, 3.87 (2 s, 3H, COO*Me*), 4.33 (m, 1H, C*H*N), 4.87 (m, 1H, C*H*COOtBu), 4.98 (m, 2H, C*H*₂=CH), 5.81 (m, 1H, CH₂=C*H*). ¹³C NMR (50.3 MHz, CDCl₃) (mixture of conformers): δ 171.3, 170.2, 162.1, 158.3, 137.8, 137.2, 115.6, 115.0, 82.4, 81.9, 61.5, 60.3, 59.5, 58.7, 52.8, 52.6, 34.9, 31.6, 30.8, 29.5, 28.4, 28.0, 27.9, 26.7, 26.4. FAB⁺MS: calculated C₁₆H₂₅NO₅ 311.17, found 312 [M+1]⁺. Calculated elemental analysis C₁₆H₂₅NO₅: C 61.72, H 8.09, N 4.50; found C 61.89, H 7.99, N 4.65.

Methyl ester **12**: obtained as a white colourless solid according to general procedure E (76% yield). m.p. = 55-56°C. [α]_{D}²² = -35.3 (*c* = 1.00, CHCl₃). ¹H NMR (200 MHz, CDCl₃) (mixture of conformers): δ 1.43, 1.44 (2 s, 9H, COO*tBu),* 1.65-2.65 (5H), 2.79 (m, 1H, CH*H*CH=CH₂), 3.78, 3.83 (2 s, 3H, COO*Me*), 4.19, 4.33, 4.69 (3 m, 2H, C*H*NCHCO, C*H*COOtBu), 5.08 (m, 2H, C*H*₂=CH), 5.72 (m, 1H, CH₂=C*H*). ¹³C NMR (50.3 MHz, CDCl₃) (mixture of conformers): δ 170.8, 170.3, 161.8, 157.9, 134.6, 134.3, 133.8, 118.1, 117.7, 117.4, 82.0, 81.6, 61.6, 60.7, 59.3, 59.1, 59.0, 52.6, 39.2, 39.0, 36.5, 36.2, 29.5, 29.2, 29.0, 27.8, 26.6. FAB⁺MS: calculated C₁₅H₂₃NO₅ 297.16, found 298 [M+1]⁺. Calculated elemental analysis C₁₅H₂₃NO₅: C 60.59, H 7.80, N 4.71; found C 60.74, H 7.77, N 4.64.

Methyl ester **13:** obtained as a colourless oil according to general procedure E (79% yield, 2 steps). [α]_{D}²² = -35.8 (*c* = 1.00, CHCl₃). ¹H NMR (200 MHz, CDCl₃) (mixture of conformers): δ 1.44 (s, 9H, COO*tBu),* 1.60-2.35 (8H), 3.79, 3.84 (2 s, 3H, COO*Me*), 4.21 (m, 1H, C*H*NCHCO), 4.36, 4.67 (2 m, 1H, C*H*COOtBu), 4.96 (m, 2H, C*H*₂=CH), 5.80 (m, 1H, CH₂=C*H*). ¹³C NMR (50.3 MHz, CDCl₃) (mixture of conformers): δ 170.9, 170.3, 162.2, 161.9, 158.4, 157.9, 137.7, 137.2, 115.2, 114.7, 82.0, 81.6, 61.3, 60.5, 59.5, 59.0, 57.4, 52.6, 34.0, 31.5, 30.5, 29.7, 29.3, 28.3, 27.8, 26.8. FAB⁺MS: calculated C₁₆H₂₅NO₅ 311.17, found 312 [M+1]⁺. Calculated elemental analysis C₁₆H₂₅NO₅: C 61.72, H 8.09, N 4.50; found C 61.60, H 8.19, N 4.40.

### Example 6: Reduction of the methyl ester.

To a solution of methyl ester **10, 11, 12** or **13** (0.230 mmol) in anhydrous THF (2.3 ml) at 0°C is slowly added a 2M solution of LiBH₄ (138 µl, 0.276 mmol). After 10 minutes, a saturated solution of NH₄Cl (2.3 ml) is added and the mixture is extracted with AcOEt (3x2.5 ml). The organic phases are dried over Na₂SO₄ and evaporated; the crude product is purified by flash chromatography (hexane/AcOEt 7:3) to give the alcohols **14,15,16** or **17.**
Alcohol **14**: obtained as a colourless oil according to general procedure F (84% yield). [α]_{D}²² = -85.7 (*c* = 1.60, CHCl₃). ¹H NMR (400 MHz, CDCl₃) (mixture of conformers): δ 1.47 (s, 9H, COO*tBu*), 1.78-2.31 (5H), 2.70 (m, 1H), 3.85, 4.35 (2 m, 1H, C*H*N), 3.86, 4.03, 4.25 (3 m, 2H, C*H*₂OH), 4.12, 4.42 (2 m, 1H, C*H*COOtBu), 5.11 (m, 2H, C*H*₂=CH), 5.75 (m, 1H, CH₂=C*H*). ¹³C NMR (75.4 MHz, CDCl₃) (mixture of conformers): δ 170.8, 170.7, 170.5, 134.5, 133.3, 118.5, 117.6, 82.5, 81.3, 60.5, 60.3, 59.1, 58.3, 56.6, 39.1, 36.8, 29.5, 29.1, 28.1, 27.8, 27.2, 26.3, 25.8. FAB⁺MS: calculated C₁₄H₂₃NO₄ 269.16, found 270 [M+1]⁺. Calculated elemental analysis C₁₄H₂₃NO₄: C 62.43, H 8.61, N 5.20; found C 62.60, H 8.55, N 5.01.
Alcohol **15**: obtained as a colourless oil according to general procedure F (90% yield). [α]_{D}²² = -84.9 (*c* = 1.00, CHCl₃). ¹H NMR (200 MHz, CDCl₃) (mixture of conformers): Conformer A δ 1.46 (s, 9H, COO*tBu*), 1.78-2.42 (8H), 3.43 (m, 1H, O*H*), 3.81 (m, 1H, C*H*N), 4.08 (m, 1H, C*H*COOtBu), 4.19 (s, 1H, C*H*₂OH), 5.02 (m, 2H, CH=C*H*₂), 5.77 (m, 1H, C*H*=CH₂). Conformer B δ 1.46 (s, 9H, COO*tBu),* 1.78-2.42 (8H), 3.48 (m, 1H, O*H*), 3.81 (m, 1H, C*H*N), 3.88 (dd, 1H, J=, J=, Hz, *H*CHOH), 4.08 (dd, 1H, J=, J=, Hz, HC*H*OH), 4.42 (m, 1H, C*H*COOtBu), 5.02 (m, 2H, CH=C*H*₂), 5.77 (m, 1H, C*H*=CH₂). ¹³C NMR (50.3 MHz, CDCl₃): δ 171.0, 170.9, 170.7, 137.8, 136.7, 116.1, 115.0, 82.7, 81.5, 60.7, 60.6, 60.2, 59.1, 58.9, 56.6, 34.0, 31.7, 30.9, 30.8, 29.4, 28.3, 28.0, 27.9, 26.6, 26.3. FAB⁺MS: calculated C₁₅H₂₅NO₄ 283.18, found 284 [M+1]⁺. Calculated elemental analysis C₁₅H₂₅NO₄: C 63.58, H 8.89, N 4.94; found C 63.70, H 8.75, N 5.00.
Alcohol **16**: obtained as a colourless oil according to general procedure F (75% yield). [α]_{D}²² = -48.7 (*c* = 1.00, CHCl₃). ¹H NMR (200 MHz, CDCl₃) (mixture of conformers): δ 1.45 (s, 9H, COO*tBu),* 1.63-2.45, 2.82 (6H), 3.44 (m, 1H, O*H*), 3.65-4.42 (4H, C*H*NCHCO, C*H*COOtBu, C*H*₂OH), 5.08 (m, 2H, C*H*₂=CH), 5.77 (m, 1H, CH₂=C*H*). ¹³C NMR (50.3 MHz, CDCl₃) (mixture of conformers): δ 171.3, 171.1, 170.8, 170.6, 134.8, 134.1, 118.6, 117.5, 82.9, 82.5, 60.8, 60.6, 60.6, 59.6, 59.1, 57.5, 38.8, 38.0, 29.9, 29.7, 28.3, 28.1, 28.0, 26.8. FAB⁺MS: calculated C₁₄H₂₃NO₄ 269.16, found 270 [M+1]⁺. Calculated elemental analysis C₁₄H₂₃NO₄: C 62.43, H 8.61, N 5.20; found C 62.25, H 8.77, N 5.26.
Alcohol **17**: obtained as a colourless oil according to general procedure F (76% yield). [α]_{D}²² = -43.1 (*c* = 1.00, CHCl₃). ¹H NMR (200 MHz, CDCl₃) (mixture of conformers): δ 1.46 (s, 9H, COO*tBu*), 1.6-2.4 (8H), 3.48 (sb, 1H, O*H*), 3.6-4.4 (4H, C*H*NCHCO, C*H*COOtBu, C*H*₂OH), 5.00 (m, 2H, C*H*₂=CH), 5.80 (m, 1H, CH₂=C*H*). ¹³C NMR (50.3 MHz, CDCl₃) (mixture of conformers): δ 171.3, 171.0, 170.8, 170.5, 138.1, 137.0, 116.0, 114.9, 82.7, 81.6, 60.6, 60.5, 60.4, 59.4, 59.3, 57.2, 33.4, 32.9, 30.8, 30.7, 29.9, 29.8, 29.7, 28.9, 28.1, 28.0, 26.9. FAB⁺MS: calculated C₁₅H₂₅NO₄ 283.18, found 284 [M+1]⁺. Calculated elemental analysis C₁₅H₂₅NO₄: C 63.58, H 8.89, N 4.94; found C 63.75, H 8.92, N 4.80.

### Example 7: Swern oxidation and intramolecular cycloaddition

To a solution of (COCl)₂ (87 µl, 1.014 mmol) in anhydrous CH₂Cl₂ (1.7 ml), maintained under a nitrogen atmosphere and cooled to -60°C, is added DMSO (96 µl, 1.352 mmol) and, following 10 minutes, a solution of alcohol **14, 15, 16** or **17** (0.338 mmol) in anhydrous CH₂Cl₂ (1.7 ml). Anhydrous TEA (377 µl, 2.704 mmol) is added after 15 minutes and the temperature allowed to rise slowly. The reaction is quenched with phosphate buffer pH 7 (3.4 ml) at 0°C and extracted with CH₂Cl₂ (3x3.5 ml). The organic phase is dried over Na₂SO₄, the solvent evaporated and the crude product thus obtained is purified by flash chromatography (hexane/AcOEt 6:4) to give the corresponding aldehydes.

To a solution of aldehyde (0.338 mmol) in EtOH/H₂O 9:1 (3.4 ml) are added NaHCO₃ (127.8 mg, 1.521 mmol) and N-benzyl hydroxylamine hydrochloride (161.8 mg, 1.014 mmol). The mixture is heated to 80°C for 1hour then the solvent is evaporated under reduced pressure. The crude product is purified by flash chromatography (hexane/AcOEt 6:4) to give the tricyclic lactams **18,19, 20, 21.** Lactam **18**: obtained as a white solid according to general procedure G (85% yield, 2 steps). M.p = 175 °C. [α]_{D}²² = +9.4 (*c* = 1.00, CHCl₃). ¹H-NMR (400 MHz, CDCl₃): δ 1.49 (s, 9H, COO*tBu*), 1.50-1.65 (m, 2H, -CH₂HC*H*CHN, - COCHCHHCH-), 1.83 (m, 1H, -*H*CHCHCOOtBu), 2.12 (m, 1H, -COCHCHHC*H*-), 2.20 (m, 1H, -CH₂*H*CHCHN), 2.40 (m, 1H, -HC*H*CHCOOtBu), 2.98 (m, 1H, C*H*CH₂O), 3.36 (d, J = 8 Hz, 1H, C*H*NBn), 3.62 (m, 1H, HCHO), 3.78 (m, 1H, C*H*NCO), 3.93 (d, 1H, J = 14.3, NC*H*₂Ph), 4.15 (dd, 1H, J1 = J2 = 7.7 Hz, HCHO), 4.48 (dd, 1H, J1 = J2 = 8.64 Hz, CHCOOt-Bu), 4.82 (d, 1H, J = 14.3, NC*H*₂Ph), 7.23-7.45 (m, 5H, Ar). ¹³C NMR HETCOR (100.6 MHz, CDCl₃): δ 129.7, 128.4, 127.3, 71.3, 65.0, 63.0, 59.3, 59.1, 42.4, 32.8, 32.9, 33.0, 28.5, 28.4. FAB⁺MS: calculated C₂₁H₂₈N₂O₄. 372.20, found 373 [M+1]⁺. Calculated elemental analysis C₂₁H₂₈N₂O₄: C 67.72, H 7.58, N 7.52; found C 67.87, H 7.66, N 7.28.
Lactam **19a**: obtained as a solid according to general procedure G (37% yield, 2 steps). M.p = 123 °C. [α]_{D}²² = +70.6 (*c* = 1.00, CHCl₃). ¹H-NMR (400 MHz, CDCl₃): δ 1.47 (s, 9H, COOt*Bu),* 1.54 (m, 1H, -COCHCHCH₂HC*H*-), 1.71 (m, 1H, COCHCHHC*H*), 1.72 (m, 1H, COCHCHCH₂*H*CH-), 1.74 (-*H*CHCH₂CHCOOtBu), 1.95 (-*H*CHCHCOOtBu), 2.06 (m, 1H, COCHCH*H*CH), 2.17 (-HC*H*CHCOOtBu), 2.35 (-HC*H*CH₂CHCOOtBu), 2.63 (m, 1H, C*H*CH₂O), 3.26 (d, 1H, J = 10.19 Hz, COC*H*N), 3.47 (dd, 1H, J1 = J2 = 6.4 Hz, HC*H*O), 3.69 (d, 1H, J = 13.88, C*H*₂Ph), 4.00 (m, 1H, C*H*NCO), 4.10 (dd, 1H, J1 = J2 = 6.4 Hz, *H*CHO), 4.50 (d, 1H, J = 13.88, C*H*₂Ph), 4.54 (dd, 1H, J = 10,23 Hz, J = 2.34 Hz, C*H*COOt-Bu), 7.20-7.46 (m, 5H, Ar). ¹³C NMR (50.3 MHz, CDCl₃): δ 171.3, 168.9, 137.4, 129.5, 129.4, 128.3, 127.3, 81.5, 73.0, 71.6, 61.2, 58.6, 45.1, 34.8, 32.7, 32.2, 29.8, 28.1, 27.2. FAB⁺MS: calculated C₂₂H₃₀N₂O₄ 386.22, found 387 [M+1]⁺. Calculated elemental analysis C₂₂H₃₀N₂O₄: C 68.37, H 7.82, N 7.25; found C 68.45, H 7.99, N 7.06.
Besides **19a**, the diastereoisomer **19b** is obtained (18% yield, 2 steps). [α]_{D}²² = - 12.0 (*c* = 1.00, CHCl₃). ¹H-NMR (400 MHz, CDCl₃): δ 1.48 (s, 9H, COO*tBu*), *1.53* (m, 1H, -COCHCHCH₂HC*H*-), 1.61 (m, 2H, COCHCHC*H₂*), 1.73 (m, 1H, - *H*CHCHCOOtBu), 1.98 (m, 1H, -HC*H*CHCOOtBu), 2.21 (m, 2H, - C*H₂*CHCOOtBu), 2.25 (m, 1H, -COCHCHCH₂HCH-), 2.68 (m, 1H, C*H*CH₂O), 3.42 (dd, 1H, J 1 = J2 = 9.09, HCHO), 3.64 (d, 1H, J = 10.58 Hz, COC*H*N), 3.82 (d, 1H, J =13.90 Hz, HC*H*Ph), 4.05 (m, 1H, C*H*NCO), 4.07 (dd, 1H, J 1 = J2 = 9.09, HCHO), 4.53 (m, 1H, CHCOOt-Bu), 4.57 (d, 1H, J = 13.90 Hz, *H*CHPh), 7.20-7.50 (m, 5H, Ar). ¹³C NMR (50.3 MHz, CDCl₃): δ 171.2, 167.4, 137.8, 129.4, 128.3, 127.2, 81.5, 71.4, 70.2, 62.3, 59.8, 54.9, 42.5, 31.1, 29.9, 29.8, 29.5, 28.2, 26.7, 25.0, 24.9. FAB⁺MS: calculated C₂₂H₃₀N₂O₄ 386.22, found 387 [M+1]⁺. Calculated elemental analysis C₂₂H₃₀N₂O₄: C 68.37, H 7.82, N 7.25; found C 68.45, H 7.99, N 7.06.
Lactam **20**: obtained according to general procedure G (74% yield, 2 steps). [α]_{D}²² = -123.1 (*c* = 1.03, CHCl₃). ¹H-NMR (400 MHz, CDCl₃): δ 1.49 (s, 9H, COO*tBu),* 1.62 (m, 1H), 1.88 (m, 1H), 2.0-2.2 (4H), 2.95 (m, 1H, C*H*CH₂ON), 3.22 (d, 1H, J = 9.0 Hz, C*H*NCH₂Ph), 3.58 (dd, 1H, J = 8.0 Hz, J = 6.1 Hz, CHC*H*HON), 3.66 (m, 1H, CHNCO), 3.92 (d, 1H, J = 14.3 Hz, CH*H*Ph), 4.17 (dd, 1H, J = 8.0 Hz, J = 8.0 Hz, CHCH*H*ON), 4.34 (dd, 1H, J = 8.6 Hz, J < 1 Hz, C*H*COOtBu), 4.90 (d, 1H, J = 14.3 Hz, C*H*HPh), 7.30 (m, 3H, *Ph*), 7.45 (m, 2H, *Ph*). ¹³C NMR (75.4 MHz, CDCl₃): δ 170.8, 166.3, 137.8, 129.3, 128.0, 126.9, 81.5, 64.1, 62.0, 59.7, 59.1, 42.6, 33.0, 31.2, 29.7, 28.5, 27.9. FAB⁺MS: calculated C₂₁H₂₈N₂O₄ 372.20, found 373 [M+1]⁺. Calculated elemental analysis C₂₁H₂₈N₂O_{4:} C 67.72, H 7.58, N 7.52; found C 67.89, H 7.56, N 7.44.
Lactam **21**: obtained as a white solid according to general procedure G (75% yield, 2 steps). m.p. = 140-142°C. [α]_{D}²² = -146.0 (*c* = 1.03, CHCl₃). ¹H-NMR (400 MHz, CDCl₃): δ 1.49 (s, 9H, COO*tBu*), 1.63-1.94 (4H), 1.98-2.12 (3H), 2.26 (m, 1H), 2.79 (m, 1H, C*H*CH₂ON), 3.17 (d, 1H, J = 10.1 Hz, C*H*NCH₂Ph), 3.52 (dd, 1H, J = 7.6 Hz, J = 6.6 Hz, CHC*H*HON), 3.67 (d, 1H, J = 13.6 Hz, CH*H*Ph), 3.85 (m, 1H, C*H*NCO), 4.14 (dd, 1H, J = 8.5 Hz, J = 7.6 Hz, CHCH*H*ON), 4.47 (d, 1H, J = 13.6 Hz, C*H*HPh), 4.65 (dd, 1H, J = 7.4 Hz, J = 3.6 Hz, C*H*COOtBu), 7.28 (m, 3H, *Ph*), 7.45 (m, 2H, *Ph*). ¹³C NMR (75.4 MHz, CDCl₃): δ 171.2, 168.5, 137.3, 129.3, 128.1, 127.1, 81.4, 73.2, 71.4, 61.4, 60.7, 58.8, 44.9, 34.0, 33.2, 32.2, 29.7, 28.0, 27.6. FAB⁺MS: calculated C₂₂H₃₀N₂O₄ 386.22, found 387 [M+1]⁺. Calculated elemental analysis C₂₂H₃₀N₂O₄: C 68.37, H 7.82, N 7.25; found C 68.45, H 7.84, N 7.06.

### Example 8: Hydrogenolysis.

A solution of lactams **18, 19a, 20** or **21** (0.10 mmol) in MeOH (1.0 ml) containing a catalytic quantity of 10% Pd/C, is stirred overnight in an atmosphere of hydrogen. The catalyst is then removed by filtration through Celite and washed with MeOH. The solvent is evaporated under reduced pressure and the crude product thus obtained is used without any further purification, giving the aminoalcohols **22, 23, 24** or **25**.
Lactam **22**: obtained as a colourless oil according to general procedure H (98% yield). ¹H-NMR (400 MHz, CDCl₃): δ 1.48 (s, 9H, COO*tBu),* 1.55-1.93 (m, 3H, - HC*H*CHCOOtBu, HCHCHN, HCHCHCHCO), 2.12 (m, 1H, HCHCHCHCO), 2.30-2.50 (m, 2 H, -*H*CHCHCOOtBu, HC*H*CHN), 2.70 (m, 1 H, -CHCHCO), 3.72 (m, 1 H, *H*CHOH), 3.93 (m, 1 H, HC*H*OH), 4.25 (m, 1 H, CHCO), 4.42 (m, 1 H, C*H*N), 4.60 (m, 1 H, CHCOOtBu). ¹³C NMR (50.3 MHz, CDCl₃): δ 171.5, 170.8, 168.1, 82.0, 70.3, 59.8, 58.9, 55.5, 32.9, 28.3, 28.1, 27.7. FAB⁺MS: calculated C₁₄H₂₄N₂O₄ 284.17, found 285 [M+1]⁺.
Lactam **23**: obtained as a colourless oil according to general procedure H (90% yield). ¹H-NMR (200 MHz, CDCl₃): δ 1.42 (s, 9H, COO*tBu),* 1.50-2.40 (m, 9H), 3.53 (m, 1H, CHCO), 3.58 (m, 2H, C*H*₂OH), 3.89 (m, 1H, C*H*N), 4.46 (m, 1H, C*H*COOtBu). ¹³C NMR (50.3 MHz, CDCl₃): δ 171.9, 171.0, 81.2, 67.9, 61.1, 58.2, 55.6, 41.4, 33.4, 32.2, 31.5, 28.1, 26.9. FAB⁺MS: calculated C₁₅H₂₆N₂O₄ 298.19, found 299 [M+1]⁺.
Lactam **24**: obtained as a colourless oil according to general procedure H (quantitative yield). ¹H-NMR (200 MHz, CDCl₃): δ 1.46 (s, 9H, COO*tBu*), 1.67 (m, 2H), 1.93-2.32 (4H), 2.57 (m, 1H, C*H*CH₂OH), 3.65 (m, 1H, C*H*NCHCO), 3.73 (d, 2H, J = 5.5 Hz, C*H*₂OH), 3.86 (db, 1H, J = 4.3 Hz, C*H*NH₂), 4.27 (sb, 3H, N*H*₂, O*H*), 4.36 (dd, 1H, J = 8.4 Hz, J < 1 Hz, C*H*COOtBu). ¹³C NMR (50.3 MHz, CDC1₃): δ 171.0, 169.9, 82.0, 65.5, 59.3, 56.9, 53.3, 38.0, 32.0, 30.7, 29.8, 29.1, 28.1. FAB⁺MS: calculated C₁₄H₂₄N₂O₄ 284.17, found 285 [M+1]⁺.
Lactam **25:** obtained as a colourless oil according to general procedure H (90% yield). ¹H-NMR (200 MHz, CDCl₃): δ 1.50 (s, 9H, COO*tBu*), 1.58-2.38 (9H), 3.30-3.85 (7H, C*H*N, PhC*H*₂OH, C*H*NH₂, N*H*₂, O*H*), 4.42 (m, 1H, C*H*COOtBu). ¹³C NMR (50.3 MHz, CDCl₃): δ 173.0, 171.1, 81.4, 68.0, 61.6, 58.3, 58.0, 40.8, 33.5, 32.9, 32.7, 32.0, 29.6, 27.9, 27.7. FAB⁺MS: calculated C₁₅H₂₆N₂O₄ 298.19, found 299 [M+1]⁺.

## Claims

1. Compounds of general formula (I): 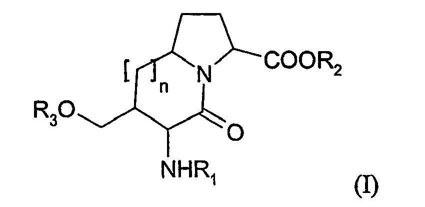 wherein:
n is 1 or 2,
R₁ is H, (C₁-C₄)alkyl, benzyl or a protective group;
R₂ is H or a protective group;
R₃ is H or heteroalkyl or a protective group;
their salts, racemic mixtures, individual enantiomers, individual diastereoisomers and mixtures thereof in any proportion.

2. The compounds according to claim 1, of general formulae (Ia) and (Ib): 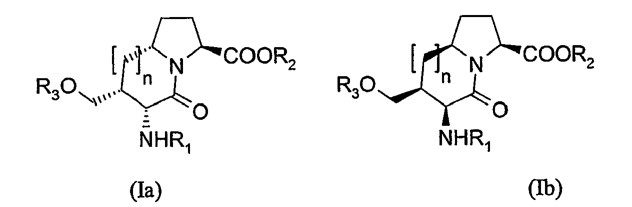 wherein n, R₁, R₂ and R₃ are as defined in claim 1 and the wedge-shaped and dotted bonds indicate that the substituents are positioned above and below the plane respectively.

3. The compound according to claims 1 or 2 **characterised in that** n is 1, R₁ is H, R₂ is tert-butyl, R₃ is H.

4. The compound according to claims 1 or 2 **characterised in that** n is 2, R₁ is H, R₂ is tert-butyl, R₃ is H.

5. The compound according to claims 1 or 2 **characterised in that** the heteroalkyl comprises an alkyl chain selected from -(CH₂)ₘCH₂OH, -(CH₂)ₘCH₂NH₂ and -(CH₂)ₘCH₂SH, wherein m is number between 0 and 10.

6. The compound according to claims 1 or 2 **characterised in that** R₁ is benzyl.

7. A process for the preparation of the compounds of formulae (I), (Ia) and (Ib) according to claims 1 to 6, **characterised by** condensing the chloride salt of oxalic acid monomethyl ester onto a compound having the following formula 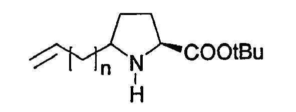 wherein n is 1 or 2, chemoselectively reducing the methyl ester group to alcohol, oxidising it to aldehyde, transforming the aldehyde into nitrone by reaction with N-benzylhydroxylamine, condensing by intramolecular 1,3-dipolar cycloaddition with the double bond present, and finally catalytically hydrogenating and isolating the compounds of general formulae (I), (Ia) and (Ib), which are optionally transformed into a salt thereof.

8. Use of the compounds of general formulae (I), (Ia) and (Ib) according to claims 1 to 6 as "reverse-turn" inducers and as conformationally constrained analogues of the homoSer-Pro dipeptide unit.

9. The use of the compounds of general formulae (I), (Ia) and (Ib) according to claims 1 to 6, as synthetic intermediates in the preparation of biologically active products.

10. The use according to claim 9 for the preparation of cyclic peptides.

11. The use according to claim 10 for the preparation of cyclic peptides containing the sequence Arg-Gly-Asp.

12. Compounds of general formula (II): 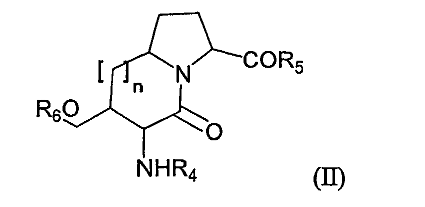 wherein:
n is 1 or 2,
R₄ and R₅ together form the sequence Asp-Gly-Arg,
R6 is H or heteroalkyl or a protective group,
their salts, racemic mixtures, individual enantiomers, individual diastereoisomers and mixtures thereof in any proportion.

13. The compounds according to claim 12, of general formulae (IIa) and (IIb) 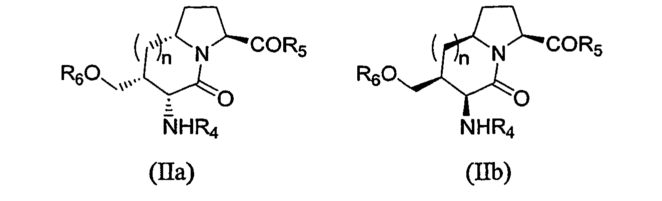 wherein n, R₄, R₅ and R₆ are as defined in claim 12 and the wedge-shaped and dotted bonds indicate that the substituents are positioned above and below the plane respectively.

14. The compounds according to claims 12 or 13 having the structure:

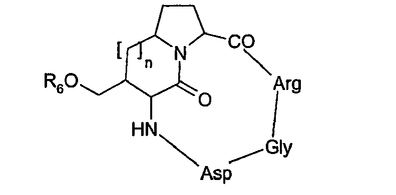

15. The compounds according to claims 12 to 13 wherein R₆ is H.

16. The compounds according to claims 12 to 13 wherein R₆ is selected from -(CH₂)ₘCH₂OH, -(CH₂)ₘCH₂NH₂ and -(CH₂)ₘCH₂SH, wherein m is number between 0 and 10.

17. The compounds of claims 12 to 16 for use as drugs.

18. The use of the compounds of claims 12 to 16 for the preparation of drugs, antagonistic towards the αvβ3 and αvβ5 integrins.

19. The use of compounds of claims 12 to 16 for the preparation of drugs with antiangiogenic activity.

20. The use according to claim 18 for the preparation of drugs intended for the treatment and/or the prophylaxis of altered angiogenic processes, metastasised tumour processes, retinopathies, acute renal damage and osteoporosis.

21. The use of the compounds of claims 12 to 16 as drug carriers.

22. The compounds according to claims 12 to 16 and pharmaceutically acceptable salts thereof, conjugated to drugs through the R₆ substituent.

23. The compounds according to claim 22 conjugated to cytotoxic and antitumour drugs.

24. A pharmaceutical composition containing at least one compound of formulae (II), (IIa) or (IIb), or pharmaceutically acceptable salts thereof, according to claims 12 to 16, as active ingredient, optionally in combination with one or more pharmaceutically acceptable carriers or excipients.

25. A pharmaceutical composition containing at least one compound of claims 22 or 23 as active ingredient, optionally in combination with one or more pharmaceutically acceptable carriers or excipients.

26. A process for the preparation of the compounds of formulae (II), (IIa) and (IIb) as defined in claims 12 to 16 **characterised by** the following steps:
- chemoselective deprotection reaction of the carboxyl group of the compound of general formulae (I), (Ia) or (Ib) and condensation with the appropriately protected Arg-Gly dipeptide;
- chemoselective deprotection of the amino group of aza-bicycloalkane and subsequent condensation with appropriately protected aspartic acid;
- transformation of the methyl ester of glycine into the benzyl ester through a transesterification reaction, followed by the simultaneous removal of the protective group from the glycine and the amino group from the aspartic acid by catalytic hydrogenation;
- condensation agent mediated intramolecular cyclisation and subsequent deprotection of the amino acid side chain protective groups.

## Patentansprüche

1. Verbindungen der allgemeinen Formel (I): 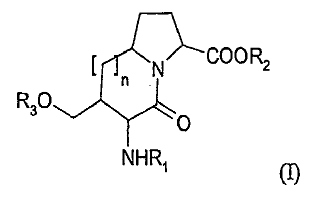 worin:
n 1 oder 2 ist,
R₁ H, (C₁-C₄)-Alkyl, Benzyl oder eine Schutzgruppe ist;
R₂ H oder eine Schutzgruppe ist;
R₃ H oder Heteroalkyl oder eine Schutzgruppe ist;
ihre Salze, racemische Mischungen, individuelle Enantiomere, individuelle Diastereoisomere und Mischungen davon in beliebigem Verhältnis.

2. Die Verbindungen gemäß Anspruch 1 der generellen Formeln (la) und (Ib): 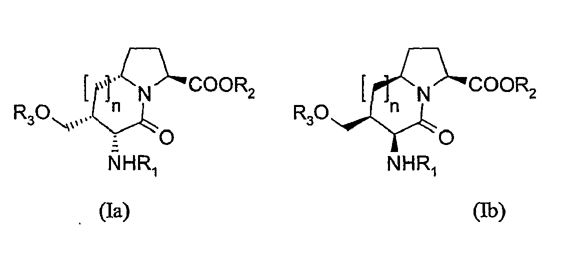 worin n, R₁, R₂ und R₃ wie in Anspruch 1 definiert sind und die keilförmigen und gestrichelten Bindungen anzeigen, dass die Substituenten oberhalb bzw. unterhalb der Ebene angeordnet sind.

3. Die Verbindung gemäß den Ansprüchen 1 oder 2, **dadurch gekennzeichnet, dass** n 1 ist, R₁ H ist, R₂ *tert*-Butyl ist, R₃ H ist.

4. Die Verbindung gemäß den Ansprüchen 1 oder 2, **dadurch gekennzeichnet, dass** n 2 ist, R₁ H ist, R₂ *tert*-Butyl ist, R₃ H ist.

5. Die Verbindung gemäß den Ansprüchen 1 oder 2, **dadurch gekennzeichnet, dass** Heteroalkyl eine Alkylkette umfasst, ausgewählt aus -(CH₂)ₘCH₂OH, -(CH₂)ₘCH₂NH₂ und -(CH₂)ₘCH₂SH, worin m eine Zahl zwischen 0 und 10 ist.

6. Die Verbindung gemäß den Ansprüchen 1 oder 2, **dadurch gekennzeichnet, dass** R₁ Benzyl ist.

7. Ein Verfahren zur Herstellung von Verbindungen der Formeln (I), (la) und (Ib) gemäß den Ansprüchen 1 bis 6, **gekennzeichnet durch** die Kondensation des Chloridsalzes von Oxalsäuremonomethylester mit einer Verbindung, welche die folgende Formel hat 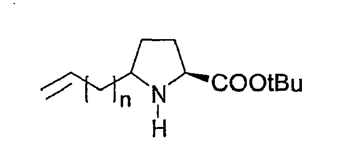 worin n 1 oder 2 ist, chemoselektive Reduktion der Methylestergruppe zum Alkohol, Oxidation desselben zum Aldehyd, Umwandlung des Aldehyds in das Nitron **durch** Reaktion mit *N*-Benzylhydroxylamin, Kondensation **durch** intramolekulare 1,3-dipolare Cycloaddition mit der vorhandenen Doppelbindung und schließlich katalytische Hydrierung und Isolierung der Verbindungen der allgemeinen Formeln (I), (la) und (Ib), welche optional in ein davon abgeleitetes Salz umgewandelt werden.

8. Die Verwendung der Verbindungen der allgemeinen Formeln (I), (Ia) und (Ib) gemäß den Ansprüchen 1 bis 6 als "Reverse Turn" -Einleiter und als konformativ eingeschränkte Analoga der homoSer-Pro Dipeptideinheit.

9. Die Verwendung der Verbindungen der allgemeinen Formeln (I), (la) und (Ib) gemäß den Ansprüchen 1 bis 6, als Syntheseintermediate in der Herstellung biologisch aktiver Produkte.

10. Die Verwendung gemäß Anspruch 9 zur Herstellung cyclischer Peptide.

11. Die Verwendung gemäß Anspruch 10 zur Herstellung cyclischer Peptide, welche die Sequenz Arg-Gly-Asp enthalten.

12. Verbindungen der allgemeinen Formel (II): 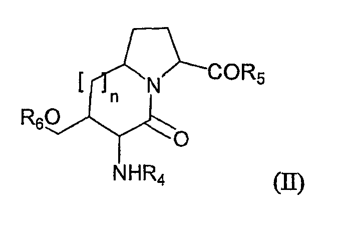 worin:
n 1 oder 2 ist,
R₄ und R₅ zusammen die Sequenz Asp-Gly-Arg bilden,
R₆ H oder Heteroalkyl oder eine Schutzgruppe ist,
ihre Salze; racemische Mischungen, individuelle Enantiomere, individuelle Diastereoisomere und Mischungen davon in beliebigem Verhältnis.

13. Die Verbindungen gemäß Anspruch 12 der allgemeinen Formel (IIa) und (IIb) 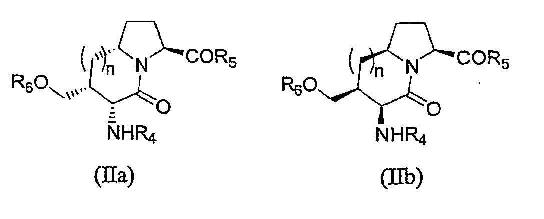 worin n, R₄, R₅ und R₆ wie in Anspruch 12 definiert sind und die keilförmigen und gestrichelten Bindungen anzeigen, dass die Substituenten oberhalb bzw. unterhalb der Ebene angeordnet sind.

14. Die Verbindungen gemäß den Ansprüchen 12 oder 13, welche die Struktur aufweisen:

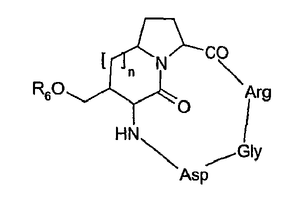

15. Die Verbindungen gemäß den Ansprüchen 12 oder 13, worin R₆ H ist.

16. Die Verbindungen gemäß den Ansprüchen 12 oder 13, worin R₆ ausgewählt ist aus -(CH₂)ₘCH₂OH, -(CH₂)ₘCH₂NH₂ und -(CH₂)ₘCH₂SH, worin m eine Zahl zwischen 0 und 10 ist.

17. Die Verbindungen gemäß den Ansprüchen 12 bis 16 zur Verwendung als Medikamente.

18. Die Verwendung der Verbindungen der Ansprüche 12 bis 16 zur Herstellung von Medikamenten, welche antagonistisch gegen αvβ3- und αvβ5- Integrine wirken.

19. Die Verwendung der Verbindungen der Ansprüche 12 bis 16 zur Herstellung von Medikamenten mit antiangiogenetischer Aktivität.

20. Die Verwendung gemäß Anspruch 18 zur Herstellung von Medikamenten, welche auf die Behandlung und/oder die Prophylaxe veränderter angiogenetischer Prozesse, metastasierter Tumorprozesse, Retinopathien, akuter Nierenschäden und von Osteoporose gerichtet sind.

21. Die Verwendung der Verbindungen der Ansprüche 12 bis 16 als Träger für Wirkstoffe.

22. Die Verbindungen gemäß den Ansprüchen 12 bis 16 und pharmazeutisch akzeptable Salze hiervon, mit Wirkstoffen verbunden durch den R₆-Substituenten.

23. Die Verbindungen gemäß Anspruch 22, verbunden mit cytotoxischen und Antitumor-Wirkstoff.

24. Eine pharmazeutische Zusammensetzung, beinhaltend wenigstens eine Verbindung der Formeln (II), (IIa) oder (IIb) oder pharmazeutisch akzeptable Salze hiervon, gemäß den Ansprüchen 12 bis 16, als aktiver Inhaltsstoff, optional in Kombination mit einem oder mehreren pharmazeutisch akzeptablen Trägern oder weiteren Hilfsstoffen.

25. Eine pharmazeutische Zusammensetzung beinhaltend wenigstens eine der Verbindungen der Ansprüche 22 oder 23 als aktiven Inhaltsstoff, optional in Kombination mit einem oder mehreren pharmazeutisch akzeptablen Trägern oder weiteren Inhaltsstoffen.

26. Ein Verfahren zur Herstellung der Verbindungen der Formeln (II), (IIa) und (IIb), wie in den Ansprüchen 12 bis 16 definiert, **gekennzeichnet, durch** die folgenden Schritte:
- Chemoselektive Entschützungsreaktion der Carboxylgruppe der Verbindungen der allgemeinen Formeln (I), (Ia) oder (Ib) und Kondensation mit einem adäquat geschützten Arg-Gly-Dipeptid;
- Chemoselektive Entschützung der Aminogruppe von Azabicycloalkan und nachfolgende Kondensation mit adäquat geschützter Asparaginsäure;
- Umwandlung des Methylesters von Glycin in den Benzylester **durch** eine Umesterungsreaktion, gefolgt von dem simultanen Entfernen der Schutzgruppe des Glycins und der Aminogruppe der Asparaginsäure **durch** katalytische Hydrierung;
- intramolekulare Cyclisierung, die **durch** ein Kondensierungsreagenz vermittelt wird und nachfolgende Entfernung der Aminosäureseitenkettenschutzgruppen.

## Revendications

1. Composés de formule générale (I) : 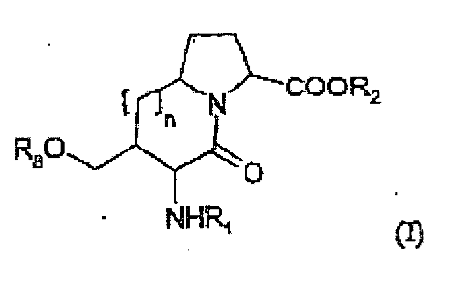 dans laquelle :
n vaut 1 ou 2,
R₁ représente H, un groupe alkyle (en C₁ à C₄), benzyle ou un groupe protecteur ;
R₂ représente H ou un groupe protecteur ;
R₃ représente H ou un groupe hétéroalkyle ou un groupe protecteur ;
leurs sels, mélanges racémiques, énantiomères individuels, diastéréo-isomères individuels et mélanges de ceux-ci dans toute proportion.

2. Composés selon la revendication 1, des formules générales (Ia) et (Ib) : 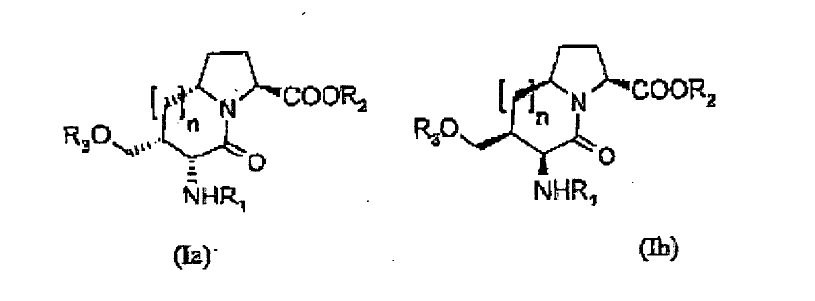 où n, R₁, R₂ et R₃ sont tels que définis dans la revendication 1 et les liaisons en forme de coin et en pointillés indiquent que les substituants sont positionnés, respectivement, au-dessus et au-dessous du plan.

3. Composé selon les revendications 1 ou 2, **caractérisé en ce que** n vaut 1, R₁ représente H, R₂ représente un groupe tert-butyle, R₃ représente H.

4. Composé selon les revendications 1 ou 2, **caractérisé en ce que** n vaut 2, R₁ représente H, R₂ représente un groupe tert-butyle, R₃ représente H.

5. Composé selon les revendications 1 ou 2, **caractérisé en ce que** le groupe hétéroalkyle comprend une chaîne alkyle choisie parmi -(CH₂)ₘCH₂OH, -(CH₂)ₘCH₂NH₂ et -(CH₂)ₘCH₂SH, où m représente un nombre entre 0 et 10.

6. Composé selon les revendications 1 ou 2, **caractérisé en ce que** R₁ représente un groupe benzyle.

7. Procédé de préparation des composés des formules (I), (Ia) et (Ib) selon les revendications 1 à 6, **caractérisé par** la condensation du sel chlorure de l'ester monométhylique de l'acide oxalique sur un composé ayant la formule suivante 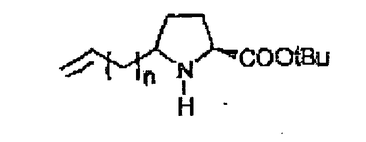 dans laquelle n vaut 1 ou 2, la réduction de manière chimiosélective du groupe ester méthylique en alcool, son oxydation en aldéhyde, la transformation de l'aldéhyde en une nitrone par réaction avec de la N-benzylhydroxylamine, la condensation par cycloaddition 1,3-dipolaire intramoléculaire avec la double liaison présente, et finalement l'hydrogénation catalytique et l'isolement des composés des formules (I), (Ia) et (Ib), qui sont éventuellement transformés en un sel de ceux-ci.

8. Utilisation des composés des formules générales (I), (Ia) et (Ib) selon les revendications 1 à 6, en tant qu'inducteurs à « rotation inverse » et en tant qu'analogues à contrainte conformationnelle de l'unité dipeptidique homoSer-Pro.

9. Utilisation des composés des formules générales (I), (Ia) et (Ib) selon les revendications 1 à 6, en tant qu'intermédiaires synthétiques dans la préparation de produits biologiquement actifs.

10. Utilisation selon la revendication 9 pour la préparation de peptides cycliques.

11. Utilisation selon la revendication 10 pour la préparation de peptides cycliques contenant la séquence Arg-Gly-Asp.

12. Composés de formule générale (II) : 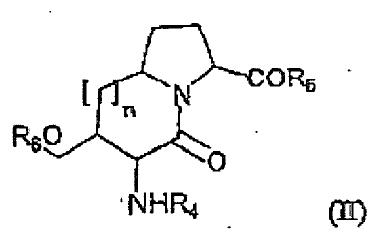 dans laquelle :
n vaut 1 ou 2,
R₄ et R₅ forment ensemble la séquence Asp-Gly-Arg,
R₆ représente H ou un groupe hétéroalkyle ou un groupe protecteur,
leurs sels, mélanges racémiques, énantiomères individuels, diastéréo-isomères individuels et mélanges de ceux-ci dans toute proportion.

13. Composés selon la revendication 12, des formules générales (IIa) et (IIb) 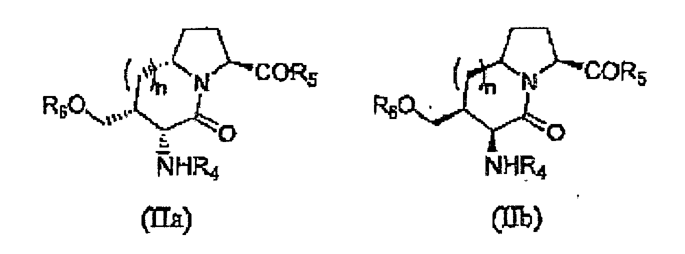 où n, R₄, R₅ et R₆ sont tels que définis dans la revendication 12 et les liaisons en forme de coin et en pointillés indiquent que les substituants sont positionnés, respectivement, au-dessus et au-dessous du plan.

14. Composés selon les revendications 12 ou 13 ayant la structure :

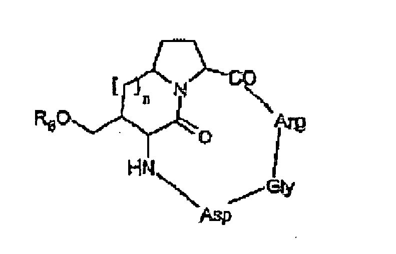

15. Composés selon les revendications 12 à 13, dans lesquels R₆ représente H.

16. Composés selon les revendications 12 à 13, dans lesquels R₆ est choisi parmi -(CH₂)ₘCH₂OH, -(CH₂)ₘCH₂NH₂ et -(CH₂)ₘCH₂SH, où m représente un nombre entre 0 et 10.

17. Composés selon les revendications 12 à 16 en vue d'une utilisation en tant que médicaments.

18. Utilisation des composés selon les revendications 12 à 16 pour la préparation de médicaments, antagonistes envers les intégrines αvβ3 et αvβ5.

19. Utilisation des composés selon les revendications 12 à 16 pour la préparation de médicaments possédant une activité antiangiogénique.

20. Utilisation selon la revendication 18 pour la préparation de médicaments destinés au traitement et/ou à la prophylaxie de processus angiogéniques modifiés, de processus tumoraux métastasés, de rétinopathies, d'une lésion rénale aiguë et de l'ostéoporose.

21. Utilisation des composés selon les revendications 12 à 16 en tant que supports de médicament.

22. Composés selon les revendications 12 à 16 et sels pharmaceutiquement acceptables de ceux-ci, conjugués à des médicaments par l'intermédiaire du substituant R₆.

23. Composés selon la revendication 22 conjugués à des médicaments cytotoxiques et antitumoraux.

24. Composition pharmaceutique contenant au moins un composé de formule (II), (IIa) ou (IIb), ou des sels pharmaceutiquement acceptables de celui-ci, selon les revendications 12 à 16, en tant que principe actif, éventuellement en combinaison avec un ou plusieurs supports ou excipients pharmaceutiquement acceptables.

25. Composition pharmaceutique contenant au moins un composé selon les revendications 22 ou 23 en tant que principe actif, éventuellement en combinaison avec un ou plusieurs supports ou excipients pharmaceutiquement acceptables.

26. Procédé de préparation des composés de formule (II), (IIa) et (IIb) tels que définis dans les revendications 12 à 16, **caractérisé par** les étapes suivantes :
- réaction de déprotection chimiosélective du groupe carboxyle du composé de formule générale (I), (Ia) ou (Ib) et condensation avec le dipeptide Arg-Gly protégé de manière appropriée ;
- déprotection chimiosélective du groupe amino de l'aza-bicycloalcane et condensation subséquente avec l'acide aspartique protégé de manière appropriée ;
- transformation de l'éther méthylique de glycine en ester benzylique par l'intermédiaire d'une réaction de transestérification, suivie de l'élimination simultanée du groupe protecteur de la glycine et du groupe amino de l'acide aspartique par une hydrogénation catalytique ;
- cyclisation intramoléculaire médiée par un agent de condensation et déprotection subséquente des groupes protecteurs de la chaîne latérale des acides aminés.
